# EUROPÄISCHE PATENTSCHRIFT

(11) **EP 0 784 959 B1**
(45) Veröffentlichungstag und Bekanntmachung des Hinweises auf die Patenterteilung: **24.09.2003**
(21) Anmeldenummer: 96116985.1
(22) Anmeldetag: 23.10.1996
(51) Int. Cl.: A61B 3/135

(54) **Kamerazusatz für einen Spaltlichtprojektor**
Slit projector provided with camera means
Caméra associé à un projecteur de fente

(30) Priorität: 01.12.1995 DE 19544867
(43) Veröffentlichungstag der Anmeldung: 23.07.1997
(73) Patentinhaber: GSF - Forschungszentrum für Umwelt und Gesundheit GmbH, 85764 Oberschleissheim (DE)
(72) Erfinder: Kumpfmüller, Helmut, 80634 München (DE); Roos, Hartmut, Dr., 81245 München (DE); Kellerer, Albrecht, Prof. Dr., 80335 München (DE); Egner, Peter, 89312 Günzburg (DE)

(56) Entgegenhaltungen:
- DE-A- 3 151 837
- DE-U- 9 407 854
- GB-A- 2 112 171
- OPHTHALMIC RESEARCH, Bd. 22, Nr. S.1, 1990, BASEL (CH), Seiten 3-8, XP000613874 K. SASAKI ET AL.: "The Multi-Purpose Camera: A New Anterior Eye Segment Analysis System"
- INTERDISCIPL. TOPICS GERONT., Bd. 13, 1978, BASEL (CH), Seiten 118-130, XP000613975 V. DRAGOMIRESCU ET AL.: "Development of a New Equipment for Rotating Slit Image Photography According to Scheimpflug's Principle"

## Beschreibung

Die Erfindung betrifft einen Kamerazusatz für einen Spaltlichtprojektor nach dem Oberbegriff des Patentanspruchs 1, wie er aus K. Sasaki et al.: The Multi-Purpose Camera: A New Anterior Eye Segment Analysis System, Ophthalmic Res. 1990, 22 (suppl. 1): 3-8 bekannt ist.

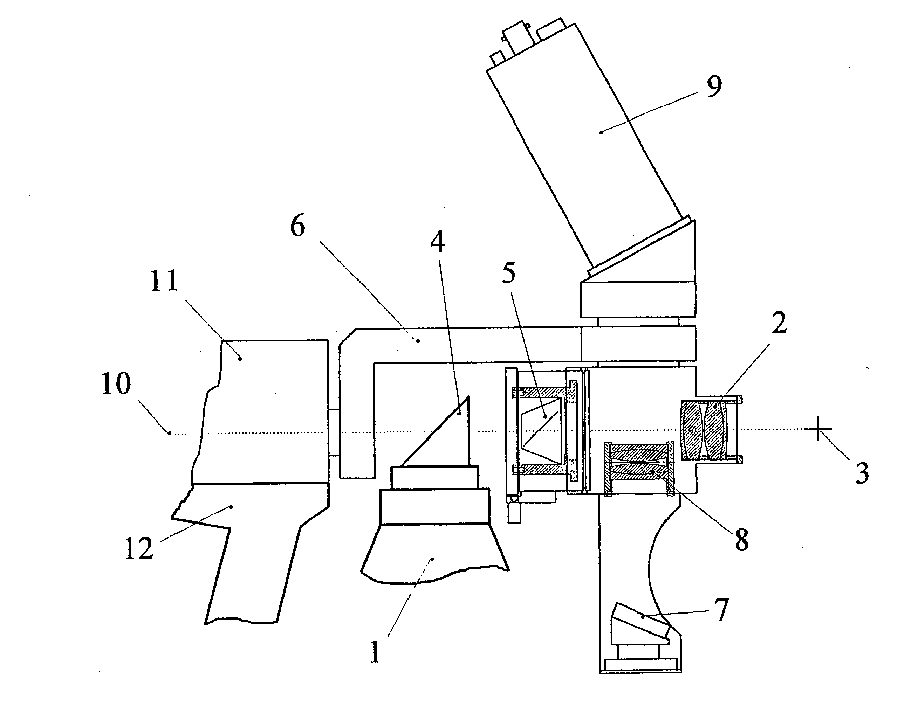

Ein Spaltlichtprojektor ist z. B. aus Ophthalmologisch-optische Instrumente, Ferdinand Enke Verlag Stuttgart 1987, S. 101, Abb. 7.1, mit Beschreibung bekannt.

Vorrichtungen der e. g. Art dienen in der Augenheilkunde dazu, quantitative Informationen über den vorderen Augenabschnitt zu gewinnen z. B. Hornhautradien, Linsenradien, Tiefe der Vorderkammer, Größe des Kammerwinkels, Entwicklung und Verlauf von Katarakten der Augenlinse, die aufgrund des normalen Alterungsprozesses entstehen oder durch spezielle Einflüsse.

Die aus V. Dragomirescu et al.: Development of a new equipment for rotating slit image photography according to Scheimpflug's principle. Interdiscipl. Topics Geront., 1978,vol. 13, 118-130, bekannte Vorrichtung muß aufwendig justiert werden und ist aufgrund der aufwendigen Strahlformung groß und unhandlich.

Ein Nachteil der Vorrichtung nach K. Sasaki besteht darin, daß bei bestimmten Einstellungen das Beobachtungsfeld durch die Augenbrauen des Patienten eingeschränkt wird.

Aufgabe der Erfindung ist es, eine Vorrichtung der e. g. Art kompakt auszugestalten ohne daß das Beobachtungsfeld eingeschränkt wird.

Gelöst wird diese Aufgabe durch die kennzeichnenden Merkmale des Patentanspruchs 1. Die Unteransprüche beschreiben vorteilhafte Ausgestaltungen der Erfindung.

Durch die leichte und kompakte Bauform der Vorrichtung werden die Lager nicht stark beansprucht. Der mechanische Aufbau kann daher kostengünstig ausgeführt werden.

Die Erfindung wird im folgenden anhand eines Ausführungsbeispiels mit Hilfe der Figur näher erläutert. Dabei zeigt die Figur schematisch einen Kamerazusatz für eine ophthalmologische Spaltlampe, wie sie standardmäßig von Augenärzten eingesetzt wird.

Das Bild des beleuchteten Spalts eines Spaltlichtprojektors 1 wird mit Hilfe einer Projektionsoptik 2 in das Auge 3 eines Patienten abgebildet. Der Spaltlichtprojektor 1 muß mit einer Aperturblende ausgestattet sein, um die nötige Tiefenschärfe für die Spaltabbildung zu gewährleisten. Dadurch entsteht in dem Auge 3 eine ausgeleuchtete Ebene. Die Projektionsoptik 2 ist an einem Halteelement 6 befestigt. Die optische Achse der Projektionsoptik 2 ist so justiert, daß sie mit der optischen Achse des Auges 3 übereinstimmt. Durch das Umlenkprisma 4 auf dem Spaltlichtprojektor 1 wird das Licht in Richtung dieser optischen Achse umgelenkt. Zwischen dem Umlenkprisma 4 und der Projektionsoptik 2 ist ein Prisma 5 (Prisma für einseitige Bildumkehr) auf dem Halteelement 6 drehbar angeordnet, mit dem das Bild des Spalts im Auge 3 senkrecht zur Zeichenebene gedreht wird. Ein Prisma vom Typ Schmidt-Pechan ist wegen seiner kompakten Bauform besonders geeignet. Andere Prismen wie z. B. nach Dove-Amici oder nach Abbe-König sind auch einsetzbar.

Bei einer weiteren Ausführungsform kann der Spaltlichtprojektor um 90° gedreht direkt in Richtung der optischen Achse angeordnet sein. Dabei wird auf das Prisma 5 verzichtet.

Am unteren Teil des Halteelements 6 ist ein Umlenkspiegel 7 befestigt. Diesem ist eine Abbildungsoptik 8 nachgeordnet, welche die im Auge 3 ausgeleuchtete Ebene über den Umlenkspiegel 7 auf den Sensor einer CCD-Kamera 9 abbildet. Die Abbildungsoptik 8 und der Umlenkspiegel 7 befinden sich auf der Seite des Halteelementes 6, welche der CCD-Kamera 9, bezüglich der Drehachse 10, gegenüber liegt. Die Abbildung erfolgt gemäß dem Scheimpflug-Prinzip, welches besagt, daß sich die Gegenstandsebene, die Objektivebene und die Bildebene (Sensor der CCD-Kamera) in einer Geraden schneiden müssen, um eine tiefenscharfe Abbildung zu erhalten. Durch folgende geometrische Parameter wird ein besonders kompakter Aufbau ermöglicht. Der Winkel zwischen der Geraden durch die Mitte des Umlenkspiegels 7 und des Auges 3 und der Drehachse 10 beträgt 45°. Der Abstand zwischen dem Umlenkspiegel 7 und dem Auge 3 beträgt ca. 110 mm. Die Neigung des Umlenkspiegels 7 ist so gewählt, daß die optische Achse der Abbildungsoptik 8 senkrecht zur Drehachse 10 verläuft und diese schneidet. Der Abstand zwischen Abbildungsoptik 8 und Auge 3 beträgt ca. 170 mm.

Das Halteelement 6 ist um eine Drehachse 10 drehbar, die mit der optischen Achse von Auge 3 und Projektionsoptik 2 übereinstimmt. Durch diese Drehung ist es möglich verschiedene Ebenen im Auge 3 zu untersuchen. Das Halteelement 6 ist in einem Träger 11 gelagert. Der Träger 11 ist an dem Mikroskopträger 12 einer ophthalmologischen Spaltlampe kraftschlüssig befestigt. Mittel zum Drehen des Halteelements 6 sind aus Gründen der Übersichtlichkeit hier nicht dargestellt. Die Drehung kann manuell über Hebel oder mit Hilfe eines Schrittmotors erfolgen. Der Drehwinkel wird über eine Skala oder elektronisch erfaßt.

Bei Drehung des Halteelements 6 um die Achse 10 um einen bestimmten Winkel dreht das Prisma 5 das Bild des Spaltes um den doppelten Winkel. Zur Synchronisation der beiden Drehungen darf das Prisma 5 nur um den halben Winkel gedreht werden. Dies kann manuell oder mit Hilfe eines Schrittmotors erfolgen.

### Bezugszeichenliste:

- 1: Spaltlichtprojektor
- 2: Projektionsoptik
- 3: Auge (Gegenstandsebene)
- 4: Umlenkprisma
- 5: Prisma
- 6: Halteelement
- 7: Umlenkspiegel
- 8: Abbildungsoptik (Objektivebene)
- 9: CCD-Kamera
- 10: Drehachse
- 11: Träger
- 12: Mikroskopträger

## Patentansprüche

1. Kamerazusatz für einen Spaltlichtprojektor, bestehend aus einem Träger, einem darin drehbar gelagerten Halteelement, einer Projektionsoptik, einer Abbildungsoptik und einer CCD-Kamera, wobei Projektionsoptik, Kamera und Abbildungsoptik an dem Halteelement befestigt sind, wobei die Anordnung von Bildebene, Gegenstandsebene und Objektivebene der Scheimpflugbedingung genügt und wobei die Drehachse des Halteelements mit der Spaltlichtachse übereinstimmt, **gekennzeichnet durch** einen Umlenkspiegel (7) zwischen Gegenstandsebene und Objektivebene, welcher auf der Seite des Halteelements (6) angeordnet ist, die der CCD-Kamera (9), bezüglich der Drehachse (10), gegenüber liegt.

2. Kamerazusatz nach Anspruch 1, **gekennzeichnet durch** ein Prisma (5) zum Drehen des Spaltbildes um die Drehachse (10).

3. Kamerazusatz nach Anspruch 1 oder 2, **gekennzeichnet durch** einen Schrittmotor zum Drehen des Halteelements (6).

4. Kamerazusatz nach einem der Ansprüche 1 bis 3, **dadurch gekennzeichnet, daß** der Träger (11) so ausgebildet ist, daß er kraftschlüssig mit dem Mikroskopträger (12) einer ophthalmologischen Spaltlampe verbunden werden kann.

5. Kamerazusatz nach einem der Ansprüche 2 bis 4, **gekennzeichnet durch** einen Schrittmotorantrieb für das Prisma (5).

## Claims

1. Camera attachment for a slit light projector, said attachment comprising a carrier, a holder which is rotatably mounted therein, a projection optical system, an image optical system and a CCD camera, the projection optical system, camera and image optical system being mounted on the holder, the disposition of the image plane, object plane and lens plane satisfying the Scheimpflug's condition, and the axis of rotation of the holder coinciding with the slit light axis, **characterised by** a deflection mirror (7) between the object plane and lens plane, which mirror is disposed on the side of the holder (6) situated opposite the CCD camera (9), with respect to the axis of rotation (10).

2. Camera attachment according to claim 1, **characterised by** a prism (5) for rotating the slit image about the axis of rotation (10).

3. Camera attachment according to claim 1 or 2, **characterised by** a stepping motor for rotating the holder (6).

4. Camera attachment according to one of claims 1 to 3, **characterised in that** the carrier (11) is so configured that it can be connected in a force-locking manner to the microscope carrier (12) of an ophthalmological slit lamp.

5. Camera attachment according to one of claims 2 to 4, **characterised by** a stepping motor drive for the prism (5).

## Revendications

1. Caméra associée à un projecteur à fente composé d'un support muni d'un élément de fixation monté à rotation, d'une optique de projection, d'une optique d'image et d'une caméra CCD, l'optique de projection, la caméra et l'optique d'image étant fixées à un élément de support,
la disposition du plan image, du plan objet et du plan de l'objectif répondant à la condition de Scheimpflug et l'axe de rotation de l'élément de fixation correspondant à l'axe de la fente,
**caractérisée par**
un miroir de renvoi (7) entre le plan objet et le plan de l'objectif, qui est prévu du côté de l'élément de fixation (6) en regard de la caméra CCD (9) par rapport à l'axe de rotation (10).

2. Caméra selon la revendication 1,
**caractérisée par**
un prisme (5) pour faire tourner la fente autour de l'axe de rotation (10).

3. Caméra selon l'une quelconque des revendications 1 ou 2,
**caractérisée par**
un moteur pas-à-pas pour faire tourner l'élément de fixation (6).

4. Caméra selon l'une quelconque des revendications 1 à 3,
**caractérisée en ce que**
le support (11) est réalisé pour être relié par une liaison de force au support du microscope (12) d'une lampe à fente ophtalmologique.

5. Caméra selon l'une quelconque des revendications 2 à 4,
**caractérisée par**
un entraînement par un moteur pas-à-pas pour le prisme (5).
